# EUROPEAN PATENT APPLICATION

(11) **EP 2 441 440 A1**
(43) Date of publication of application: **18.04.2012**
(21) Application number: 11185493.1
(22) Date of filing: 17.10.2011
(51) Int. Cl.: A61K 9/00, A61K 47/10, A61K 8/00, A61K 36/00

(54) **Natural pharmacological aid for prevention of periodontal inflammatory disease**

(30) Priority: 18.10.2010 IT MC20100101
(71) Applicant: Straccia, Luigina, 63023 Fermo (FM) (IT)
(72) Inventor: Straccia, Luigina, 63023 Fermo (FM) (IT)
(74) Representative: Cutropia, Gianluigi

(57) **Abstract**

A natural pharmacological aid for prevention of periodontal inflammatory disease is disclosed, wherein said aid is a spray containing a liquid aqueous solution suitable to be sprayed in the patient's mouth; said aqueous solution comprising the following excipients dissolved in water: Calendula, Echinacea, Alchemilla and thermo-gel agent suitable to convert said aqueous solution in gel, when the aqueous solution comes in contact with the patient's mouth at a temperature of about 36 °C.

## Description

The present patent application for industrial invention relates to a natural pharmacological aid for prevention of periodontal inflammatory disease, in particular gingivitis and chronic periodontitis.

Gingivitis is a chronic inflammation, with exclusively bacterial aetiology, affecting the marginal periodontium (gum). Gingivitis is clinically characterized by modification of color and alteration of consistency and shape of marginal gums. Gums can appear reddened, edematous, and sometimes swollen; capillary dilatation, caused by the inflammatory process, determines onset of edema and high likelihood of bleeding, both spontaneously and when using, for example, a toothbrush.

Clinical examination shows the presence of dental plaque, tartar or fragments of various types, in both radicular and interproximal marginal area. As often reported by patients, symptoms, include sensitivity to hot, spontaneous bleeding, halitosis and pain. The inflammatory injury is limited to marginal connective issue area and does not affect the bone structure. Prognosis is favorable with a suitable treatment; otherwise gingivitis evolves in periodontitis or periodontal disease.

Gingivities treatment provides for controlling etiological local factors, that is optimum control of dental plaque formation by patient (educated and motivated by dental staff), therapeutic treatments carried out in the dentist's surgery by specialized professionals, intervening with procedures like tartar ablation, tartar removal, scaling, and root-plaining.

Chronic periodontitis is an infective disease (bacterial plaque), clinically characterized by pathological involvement of all tissue components of periodontal organ (gun, periodontal ligament, alveolar bone). Such pathology can reveal itself as a simple inflammatory status, without tissue damage, or develop a destructive injury involving one or more tissues, according to severity and progress of disease.

Chronic periodontitis develops as consequence of colonization of periodontal and subgingival sites by specific bacteria. Harmful substances produced by bacteria cause the infection and destruction of tissues surrounding and supporting the dental element, causing mobility and consequent loss (defined in the past as "pyorrhea").

The pharmacological control of bacterial plaque provides for using antibacterial drugs to eliminate the pathogen bacterial flora. The following drugs are used:
- Clorexidina: it is the most studied and most used oral antiseptic, which is considered to be the most efficacious anti-plaque and anti-gingivitis chemotherapeutic available. It has bactericidal properties and is active against Gram⁺, Gram- bacteria and fungi. Its action is carried out by means of alteration of bacterial cellular wall with consequent cellular lysis. Clorexidina can bind with phosphate, sulphate and carboxyl ions, from which it frees slowly, with activity prolonged over time. It is used as mouthrinse and spray mouthrinse, also before operations, in order to reduce the number of bacteria in the oral cavity and contrast formation of bacterial plaque or onset of gingivitis; it is also used in chronic ulcerative gingivitis and prevents dental caries in smooth surfaces. According to studies and experimentations made Long (1982) the optimum concentration is 0.12%, some commercial products reach a concentration of 0.20%, whereas in association with fluorides, concentration of both is 0.05%. Collateral effects due to the use of clorexidina are brownish pigmentations on teeth and taste alteration, especially with sweet.

Listerine: it is a mouthrinse indicated as anti-plaque and gingivitis treatment, it contains menthol, thymol, methyl salicylate and eucalyptol; its antiseptic activity is due to the presence of the four essential oils dissolved in alcohol (which have no anti-bacterial action being present in concentration lower than 40%), capable of breaking plaque biofilm and penetrate the cellular wall with higher efficacy with respect to other mouthrinses for daily use.

Sanguinarina: it is an alkaloid extracted from the rhyzome of Sanguinaria Canadiensis with antibacterial and anti-halitosis activity; it fixes firmly on plaque, but tends to stain teeth with yellow-brownish pigmentations. Its action improves in the form of mouthrinse. Unfortunately, an American study has demonstrated that the use of preparations containing sanguinarina includes a risk of precancerous injuries to mouth mucosa, which is estimated to be ten times higher than other products without such an anti-bacterial.
• Antiseptic of oral cavity, hexetidine: it is a drug with antiseptic, antibacterial and antimicotic action, used as local anesthetic; as oral topical, it has antiplaque activity, it also has anti-inflammatory activity and is therefore useful as anti-exudative in all affections of the oral cavity with inflammatory component. It has a large action spectrum, including Gram⁺ and Gram- bacteria and some fungi. Its activity is of bactericidal type, with action mechanism referable to competitiveness against thiamine, indispensable growth factor for microorganisms.

Fluorine salts and fluorite paints: they have the capacity of adhering to enamel for long and release fluorine to teeth slowly. Historically, already in 1930 epidemiological studies carried out by the Public Sanitary Service of the United States and directed by Dr. Frendley noted the importance of fluorine in water for caries prevention.

However, said known drugs are impaired by some drawbacks. In order for a drug to be efficacious and correctly used, some problems must be overcome.
- how to get the drug in contact with the deeper layers of plaque;
- which bacteria must be eliminated and therefore identify the most selective drugs;
- establish the duration of treatment, avoiding undesired effects;
- guarantee sufficient adhesion of active principle to affected surfaces;
- easy self-administration.

The market does not offer products with all the aforementioned requirements; some products, possibly with wide spectrum, indifferently eliminate self and non-self microorganisms, leaving open space to onset of mycosis. In case of administration for a long time, some products may cause undesirable secondary effects, even with therapeutic doses.

In fact, bacterial plaque is composed of different microbic populations: a Gram⁺ aerobic fraction responsible for caries and a

Gram- anaerobic fraction responsible for inflammation and consequently periodontal disease. A plaque mainly formed of streptococci has no periodontolithic action, but unfortunately it has a cariogenic action and in such a case the use of a drug selective for Gram- bacteria risks to increase the incidence of caries, thus being dangerous for carioreceptive patients.

The WO2004/069278; WO02/41837; WO03/037276 and WO00/07603 patent documents disclose viscosity modifiers, such as Polaxamer and Pluronic, used in oral cavity together with anti-inflammatory substances.

WO2005/063184 discloses the use of extracts from plants, in particular calendula, for treatment of oral disease, such as gingivitis, plaque and tartar.

WO2010/045969 discloses a composition of useful substances for treatment of mucosa injuries and composed of at least two compounds chosen from Alchemilla extract, mimosa extract, and Echinacea extract.

The purpose of the present invention is to solve the drawbacks of the prior art, providing a natural pharmacological aid for prevention of periodontal inflammatory disease that is efficient, efficacious and easy to be self-administered by the patient.

This purpose has been achieved according to the invention, with characteristics claimed in independent claim 1.

Advantageous embodiments appear from the dependent claims.

According to the invention, the natural pharmacological aid for prevention of periodontal inflammatory disease, in particular gingivitis and chronic periodontitis, is composed of a spray containing a liquid aqueous solution suitable to be sprayed in the patient's mouth. Said aqueous solution comprises the following excipients dissolved in water:
- Calendula
- Echinacea
- Alchemilla
- thermo-gel agent suitable to convert said aqueous solution in gel, when the aqueous solution comes in contact with the patient's mouth at a temperature of about 36°C.

Advantageously, Calendula, Echinacea and Alchemilla are used respectively as tincture. Each tincture comprises about 30 - 50% of triturated leaves and the remaining part of alcohol. Preferably, each tincture comprises 60% of ethanol at 96 ° and 40% of triturated leaves.

The quantity of Calendula tincture is comprised between 8 - 15% with respect to the total weight of solution.

The quantity of Echinacea tincture is comprised between 8 - 15% with respect to the total weight of solution.

The quantity of Alchemilla tincture is comprised between 8 - 15% with respect to the total weight of solution.

The quantity of thermo-gel agent is comprised between 15 - 20% with respect to the total weight of solution.

In the Official Pharmacopoeia, the section about "herbs and plants that can be used by chemists in preparation of health products" includes Calendula, composed of dried flowers, either entire or cut, containing not less than 0.4% of flavonoids; the material is compliant with the European Pharmacopoeia monograph and refers to dry drug.

The chemical composition of Calendula is as follows:
1) Triterpene saponins
2) Free esterified triterpene alcohols
3) Carotenoids
4) Flavonoids
5) Polysaccharides
6) Sterols
7) Sesquiterpenoids
8) Essential oil

Experiments in vitro have demonstrated anti-microbic and anti-viral, angiogenic, anti-inflammatcory effects.

Experiments in vitro have demonstrated effects on treatment of wounds. Calendula extract is capable of favoring healing of wounds, because of two mechanisms; it stimulates the activity of fibroblasts and migrant cells and significantly increases angiogenesis at cutaneous level.

Calendula contains hydrosoluble compounds capable of favoring hyaluronan deposition and increase tissue neovascularization, with consequent anti-inflammatory, anti-edematous and anti-tumoral effects.

Toxic and secondary effects of calendula are sensitivity to members of Compositae family and weak cutaneous sensitization, but no cases of dermatitis from contact are documented.

The combination of calendula consists in a twofold action mechanism:
- desensitizing effect that reduces the level of dental sensitivity and simultaneously pain intensity; and
- anti-inflammatory and disinfectant effect that provides anti-microbic action for inflammatory disease of periodontium.

Alchemilla vulgaris is a plant of the Alchemilla genus, of the Rosaceae family. Alchemilla is an officinal plant rich in tannin and provided with astringent and antiseptic activity.

### MAIN CONSTITUENTS

1) Tannins (6-8%): ellagitannins
2) Bitter principle
3) Essential oil, phytosterols, flavonoids
4) Saponins
5) Palentic acid
6) Traces of silicic acid

Alchemilla has astringent, anti-inflammatory, healing, hemostatic properties. It is a plant known for sedative-antalgic properties, partially attributed to the presence of flavonoids. Flavonoids are also responsible for diuretic and depurative properties. In the past Alchemilla Vulgaris L. has been largely used as anti-hemorragic and vasoconstrictory and, therefore, has been used to stop bleeding and cleanse wounds.

The topical use of the plant is the typical use of tannin-containing plants: astringent, antiseptic and anti-inflammatory action. It is used in the phlogosis of oropharyngeal mucosa; gingival inflammations (gingivitis) for its anti-bacterial action (active on Staphylococcus aureus, Bacillus subtilis); mucosa irritations and inflammations.

Use in popular medicine has not demonstrated any secondary toxic effects

Alchemilla belongs to the Rosacea family and is recognizable from crenate fan-shaped leaves (from 7 to 11 lobes) covered with hair on lower side. It was traditionally used to stop internal and external bleeding.

### MAIN CONSTITUENTS

1) Tannins,
2) Ellagitannins,
3) Bitter principle,
4) Phytosterols,
5) Flavonoids,
6) Saponins,
7) Essential oil,
8) Palmitic acid
9) Stearic acid,
10) Traces of silicic acid.

Alchemilla is a progesterone-like plant and its essential oil contains: - tannins (5-7%): gallotannins, ellagitannins, catechic tannins and ellagic acid, flavonoids (0.2%): derivatives of quercetins, caempferol, cianidine, - pseudosaponin: tormentoside - triterpenes: ursolic acid - vegetal aromatic acids: caffeic acid, chlorogenic acid, ferulic acid - phytosterols - cumarine: p-cumaric acid. Dry Alchemilla extract contains tannins and flavonoids.

Alchemilla has astringent, antidiarroheic, antiseptic activity, sedative and antalgic, anti-inflammatory, spasmolitic, healing action, useful in genital affections, amenorrhea, leucorrhea, diarroheic forms, colitis, head ache, menopausa and hormonal dysfunctions.

Alchemilla is useful in mild diarroheic forms and irritable colon; the presence of flavonoids gives sedative and anthalic properties, it is useful in amenorrhea, arthralgia; flavonoids also have diuretic and depurative properties; it is also used as antihaemorragic and vasoconstrictor (for the presence of tannins and flavonoids); it is useful in fever, bruises, menopause, painful menstruation, in cases when haemostatic astringent activity is necessary, such as gastroduodenal ulcers.

Alchemilla has:
- antihaemorragic action: especially useful in case of metrorrhagia, ulcers etc. due to presence of tannins;
- antibacterial action: it is active on staphylococcus aureus and bacillus subtilis, also this action is referable to presence of tannins;
- vasoprotective action: referable to flavonoids;
- antimutagenic action: due to ellagitannins and ellagic acid;
- antiviral action: for ellagitannin content, efficacious virustatic activity, for example versus herpes zoster.

The external use of Alchemilla is used in vaginal and anal pruritus (in form of decoction). It is also useful in pharyngitis, inflammations of oral cavity, gingivitis, tooth ache, sore throat, hoarseness (gargles with decoction, 1 gram per cup), for tannins and flavonoids it is used to cleanse wounds, abrasions, leucorrhea, and it is useful in eye inflammations.

Use in popular medicine has not demonstrated any secondary toxic effects

The synergistic effect of Calendula, Echinacea and Alchemilla provides a disinfectant anti-inflammatory useful to prevent the course of gingivitis in periodontal disease. However, in order to be effective, it must ensure a sufficient permanence in gingival tissue for active principles to be absorbed and consequently act.

This function is carried out by thermo-gel agent, preferably Poloxamer® (CAS 9003-11-6), in particular Poloxamer® 407, which has the peculiarity of turning into gel when temperature changes. Such a type of thermo-gel agent is disclosed in patent US4,474,753. Alternatively, Carbopol ® 980 can be used as thermo-gel agent.

The preparation in aqueous solution, at a temperature not higher than 20 degrees, is in liquid form, therefore a solution suitable for spray is obtained at ambient temperature. As soon as such a solution comes in contact with the patient's mouth, which is approximately 36 - 37°C, it immediately turns into gel.

Another peculiarity of Poloxamer 407 is the capability of increasing absorption of active principles. The use of thermo-gelling sprays with anti-inflammatory and disinfectant active principles can replace the products (Clorexidina) already available on the market, thus guaranteeing permanence and adhesion of about 20 minutes in gingival tissues and dental surfaces (enamel, dentin, radicular cement). The use of a spray administered before the therapeutic hygiene treatment improves the inflammatory status of the periodontium, allowing the dental hygienist and dentist to operate on disinfected structures, with lower bacterial charge, lower sensitivity and lower bleeding.

In fact, the action of the aid according to the invention is carried out at multiple levels:
- anti-inflammatory action; it margins the damaging capacity of inflammatory infiltrate;
- antibacterial, antiviral action;
- antimycotic action;
- analgesic effect, as consequence or reduced inflammation;
- desensitizing action, due to presence of ethanol at 96° in Calendula, Echinacea and Alchemilla tinctures;
- healing action, it guides repair of injured tissues.

The proposed galenical is a mother tincture of calendula, alchemilia and echinacea, which are included at 10% in aqueous solution containing a suitable vehicle capable of guaranteeing desired adhesion.

Advantageously, a potassium salt is used as solution preservative and sterilizer, preferably potassium sorbate that has the function to kill microbes in solution water. The quantity of potassium salts in weight percentage is comprised between 0.1 - 0.3% of aqueous solution.

Finally, to all aforementioned excipients, a pH regulating substance is added until PH=5 is obtained. Since excipients are basic, citric acid can be used as PH regulator, for example in 5% solution.

Following is a recipe for production of 100 g of aqueous solution.

| EXCIPIENTS | Grams |
|---|---|
| Calendula Tincture | 10 g |
| Echinacea Tincture | 10 g |
| Alchemilla Tincture | 10g |
| Poloxamer407 | 17.5 g |
| Potassium sorbate | 0.2 g |
| 5% solution citric acid | Quantity sufficient to obtain ph=5 |
| Sterile water for injectable preparations | Quantity sufficient to obtain 100g of aqueous solution |

### CLINICAL EXPERIMENTATION

Experimentation was carried out on eight patients with evident signs and symptoms of acute gingivitis, with same intensity. Said symptoms are:
1) swelling of gingival tissues (gingival margin and interdental papillae);
2) spontaneous bleeding, hemorrhage;
3) red-violaceous gum color;
4) painful sensitivity to hot; and
5) painful sensitivity to cold.

### CLINIC CASE 1

Two patients were administered a mouthrinse based on clorexidina at 2%, oral gargles in morning and evening (permanence time in mouth one minute), for three days. Tissues were inspected every day. After the fourth day the following symptoms were noted:
1) light swelling of tissues
2) light bleeding
3) light red gum color
4) light sensitivity to hot
5) sensitivity to cold.

In said experiment the most popular known technique used to treat inflammations of periodontium gave unsatisfactory results. Therefore, the applicant investigated alternative solutions with natural herb extracts.

### CLINIC CASE 2

Two patients were administered an Echinacea-Alchemilla spray, morning and evening for three days. Tissues were inspected every day. After the fourth day the following symptoms were noted:
1) lack of swelling
2) light bleeding
3) light red gum color
4) light sensitivity to hot
5) sensitivity to cold.

As it was expected, due to the intrinsic properties that are known for Echinacea and Alchemilla, after four days, symptom (1) was eliminated and symptoms (2) - (4) were reduced, whereas symptom (5) persisted. Therefore, additional tests were made by the applicant in the attempt to improve results.

### CLINIC CASE 3

Two patients were administered a Calendula spray, morning and evening for three days. Tissues were inspected every day. After the fourth day the following symptoms were noted:
1) reduced swelling of gingival tissues
2) lack of bleeding
3) pink color
4) light sensitivity to hot
5) lack of painful sensitivity to cold.

Given the known healing effect of Calendula, the resolution of symptoms (2) and (3) was predictable. Instead, totally surprising was the fact that Calendula resolved the problem of sensitivity to cold. However, symptoms (2), (4) were not totally eliminated. After said tests, the composition of the invention, as described in case clinic 4, was developed.

### CLINIC CASE 4

Two patients were administered an Echinacea- Alchemilla-Calendula-Poloxamer 407 spray, morning and evening for three days (because of Poloxamer, permanence time in mouth is 10 minutes). Tissues were inspected every day. Surprisingly, after the second day of treatment, lack of bleeding and lack of painful sensitivity to hot were noted. After the fourth day the following symptoms were noted:
1) lack of swelling of gingival tissues (gingival margin and interdental papillae perfectly adhere to the tooth surface)
2) lack of bleeding (from second day)
3) coral pink gum color
4) lack of painful sensitivity to hot (from second day)
5) lack of painful sensitivity to cold (from second day)

As clearly demonstrated by said tests, natural active principles that actively work in mutual synergy have been identified in the composition of the invention, reinforcing the therapeutic effect of these substances and reducing treatment time.

## Claims

1. Natural pharmacological aid for prevention of periodontal inflammatory disease, in which said aid is a spray containing a liquid aqueous solution suitable to be sprayed in the patient's mouth; said aqueous solution comprising the following excipients dissolved in water:
- Calendula,
- Echinacea,
- Alchemilla, and
- thermo-gel agent suitable to convert said aqueous solution in gel, when the aqueous solution comes in contact with the patient's mouth at a temperature of about 36 °C.

2. Aid as claimed in claim 1, **characterized in that** said Calendula, Echinacea, Alchemilla are alcohol-based tinctures containing about 30-50% of triturated leaves and the remaining part of alcohol.

3. Aid as claimed in claim 2,, **characterized in that**:
- the quantity of Calendula tincture is comprised between 8 - 15% with respect to the total weight of solution.
- the quantity of Echinacea tincture is comprised between 8 - 15% with respect to the total weight of solution.
- the quantity of Alchemilla tincture is comprised between 8 - 15% with respect to the total weight of solution,
- the quantity of thermo-gel agent is comprised between 15 - 20% with respect to the total weight of solution.

4. Aid as claimed in any one of the preceding claims, **characterized in that** said aqueous solution also comprises a potassium salt as preservative and sterilizer of the aqueous solution.

5. Aid as claimed in claim 4, **characterized in that** the quantity of potassium salt is comprised between 0.1 - 0.3 % with respect to the total weight of solution.

6. Aid as claimed in claim 4 or 5, **characterized in that** said potassium salt comprises sorbatum potassium.

7. Aid as claimed in any one of the preceding claims, **characterized in that** said thermo-gel agent comprises Poloxamer®.

8. Aid as claimed in any one of the preceding claims, **characterized in that** said aqueous solution comprises citric acid until PH5 of the solution is obtained.

9. Use of aid as claimed in any one of the above claims, for prevention of periodontal inflammatory disease.

10. Use of aid as claimed in claim 9, for prevention of gingivitis and/or chronic periodontitis.

11. Use of aid as claimed in claim 9, for elimination of painful gum sensitivity to cold.

12. Use of aid as claimed in claim 9, for elimination of painful gum sensitivity to hot.
